# EUROPÄISCHE PATENTSCHRIFT

(11) **EP 3 765 843 B1**
(45) Veröffentlichungstag und Bekanntmachung des Hinweises auf die Patenterteilung: **12.07.2023**
(21) Anmeldenummer: 19720765.7
(22) Anmeldetag: 10.03.2019
(51) Int. Cl.: G01N 33/08, A01K 45/00, G01N 21/31

(54) **EINRICHTUNG ZUR UNTERSUCHUNG VON BRUTEIERN**
DEVICE FOR EXAMINING HATCHING EGGS
DISPOSITIF POUR INSPECTER DES OEUFS À COUVER

(30) Priorität: 11.03.2018 DE 102018001987; 11.03.2018 DE 202018001316 U
(43) Veröffentlichungstag der Anmeldung: 20.01.2021
(73) Patentinhaber: EVONTA-Technology GmbH, 01109 Dresden (DE)
(72) Erfinder: FISCHER, Björn, 09212 Limbach-Oberfrohna (DE); MEISSNER, Sven, 09618 Brand-Erbisdorf (DE)
(74) Vertreter: Kailuweit & Uhlemann Patentanwälte Partnerschaft mbB
(86) Internationale Anmeldenummer: PCT/DE2019/000065
(87) Internationale Veröffentlichungsnummer: WO 2019/174661

(56) Entgegenhaltungen:
- CH-A- 180 890
- US-A1- 2013 044 210
- US-A1- 2017 284 989
- SMITH D P ET AL: "Fertility and embryo development of broiler hatching eggs evaluated with a hyperspectral imaging and predictive modeling system", INTERNATIONAL JOURNAL OF POULTRY SCIENCE, ASIAN NETWORK FOR SCIENTIFIC INFORMATION, PK, Bd. 7, Nr. 10, 2008, Seiten 1001-1004, XP002608148, ISSN: 1682-8356
- ZHIHUI ZHU ET AL: "Nondestructive detection of infertile hatching eggs based on spectral and imaging information", INTERNATIONAL JOURNAL OF AGRICULTURAL & BIOLOGICAL ENGINEERING, Bd. 8, Nr. 4, August 2015 (2015-08), Seiten 69-76, XP055602402, DOI: 10.3965/j.ijabe.20150804.1672

## Beschreibung

Die Erfindung betrifft Einrichtungen zur Untersuchung von Bruteiern.

Zur Haltung und Aufzucht insbesondere von Vögeln ist unter anderem auch eine Bestimmung des Geschlechts von großer Bedeutung. Dabei ist insbesondere der Tierschutz einzuhalten, wobei eine Belastung von Tieren weitestgehend auszuschließen ist.

Durch die Druckschrift DE 10 2007 013 107 A1 ist ein Verfahren zur Bestimmung des Geschlechts von Vögeln offenbart, wobei ein DNA-relevantes Zellmaterial aus dem Schaft einer jungen Feder des Vogels untersucht wird. Ein durch Bestrahlung mit Licht entstehendes Spektrum wird mit Referenzspektren verglichen, so dass eine Geschlechtszuordnung des Vogels getroffen werden kann.

Durch die Druckschrift DE 10 2010 006 161 B3 ist ein Verfahren und eine Vorrichtung zur Bestimmung des Geschlechts von befruchteten und nicht bebrüteten Vogeleiern bekannt, wobei ein Ei eine feste Eischale, ein von der Eischale und weiteren Eihüllen umgebenes Eidotter und eine dem Eidotter zugeordnete Keimscheibe aufweist. Dabei wird eine Sonde zur Messung eines Spektrums durch ein Loch in der Eischale hindurch in Richtung zur Keimscheibe mit Keimscheibenzellen geführt. Mit der Sonde werden die Keimscheibenzellen spektroskopisch untersucht und das Spektrum klassifiziert. Zur Bestimmung des Geschlechts ist ein nicht unerheblicher Aufwand notwendig, wobei die Eischale gelocht werden muss.

Die Druckschrift EP 2 336 751 B1 zeigt ein Verfahren zur Bestimmung des Geschlechts an Vogeleiern, wobei mit einer Strahlungsquelle elektromagnetische Strahlung auf die Keimscheibe eines unbebrüteten Eies emittiert und nach dem Abschalten der Strahlungsquelle am bestrahlten Bereich der Keimscheibe das Abklingverhalten der durch die elektromagnetische Strahlung angeregten Eigenfluoreszenzintensität zeit- und spektralaufgelöst für mindestens eine Wellenlänge der Eigenfluoreszenz mit einem Detektor erfasst wird. Daraus wird das jeweilige Ei als weiblich oder männlich eingestuft.

Die Publikation von Smith D. P. et al. "Fertility and embryo development of broiler hatching eggs evaluated with a hyperspectral imaging and predictive modeling system" (International Journal of Poultry Science, Asian Network for Scientific Information, PK, Bd. 7, Nr. 10, 2008, Seiten 1001-1004) zeigt ein hyperspektrales Bildgebungssystem zur Bestimmung der Fruchtbarkeit und der Embryonalentwicklung von Bruteiern. Dazu werden Eier mit einer Wolfram-HalogenLampe als Quelle für elektromagnetische Strahlung durchleuchtet. Direkt über den Eiern ist ein hyperspektrales Bildgebungssystem positioniert, welches in der Lage ist, Bilder im Bereich von 400-1000 nm zu erfassen. Dabei werden zeitgleich immer vier Eier durchleuchtet. Durch einen Teiler ("divider") in X-Form wird in D1 eine Kreuzkontamination des Lichts verhindert.

Die US 2013/044210 A1 offenbart ein hyperspektrales Verfahren zum Erfassen des gegenwärtigen Zustands eines Vogeleies, bei dem ein neuronaler Netzwerkalgorithmus verwendet wird, um das Spektrum eines Testeies mit einer Referenz zu vergleichen. Dazu wird zuerst das Vogelei zunächst durch eine Lichtquelle beleuchtet, dann das Spektrum eines Vogeleies über mindestens einen vorbestimmten Wellenlängenbereich gemessen und danach ein vordefinierter Algorithmus angewandt, um anschließend das gemessene Spektrum mit einer vordefinierten Datenbank von Spektren zu vergleichen und so den gegenwärtigen Zustand des Eies zu beurteilen.

Dokument US 2017/284989 A1 beschreibt ein Ei-Identifikationssystem zur Bestimmung der Lebensfähigkeit von Eiern. Das System umfasst eine Emitteranordnung zum Emittieren elektromagnetischer Strahlung in Richtung einer Vielzahl von in deren Nähe positionierten Eiern. Dabei wird tlw. für jedes Ei eine separate Lichtquelle verwendet. Eine Detektoranordnung, welche nahe der Emitteranordnung positioniert ist, weist mehrere Detektoren auf, die in Bezug auf die Emitteranordnung fix positioniert und konfiguriert sind, um elektromagnetische Strahlung zu erfassen, die durch die Eier übertragen wird. Ferner steht ein Prozessor mit der Detektoranordnung in Verbindung und bestimmt die Lebensfähigkeit der Eier unter Verwendung der erfassten elektromagnetischen Strahlung.

Der im Patentanspruch 1 angegebenen Erfindung liegt die Aufgabe zugrunde, Bruteier zu analysieren und so Eigenschaften der Bruteier selbst und/oder der Embryonen in den Bruteiern zu bestimmen.

Diese Aufgabe wird mit den im Patentanspruch 1 aufgeführten Merkmalen gelöst.

Die Einrichtungen zur Untersuchung von Bruteiern zeichnen sich insbesondere dadurch aus, dass Eigenschaften der Bruteier selbst und/oder der Embryonen in den Bruteiern bestimmbar sind.

Dazu ist ein Träger mit beabstandet zueinander angeordneten Bruteiern zwischen einer Quelle für elektromagnetische Wellen und einer Hyperspektralkamera angeordnet, der weiterhin Ausschnitte zur Platzierung der Bruteier und lichtdichten Ankopplung der elektromagnetischen Wellen besitzt. Zwischen den Bruteiern befinden sich Trennwände. Die Quelle für elektromagnetische Wellen und die Hyperspektralkamera sind mit einem Datenverarbeitungssystem verbunden, welches aus dem Abbild der Hyperspektralkamera die Eiabbilder ermittelt und dem jeweiligen Eiabbild hyperspektrale Daten und die Position des jeweiligen Bruteies auf dem Träger zuordnet.

Die Bruteier sind Vogeleier, insbesondere bebrütete Hühnereier.

Vorteilhafte Ausgestaltungen der Erfindung sind in den Patentansprüchen 2 bis 7 angegeben.

Auf dem Träger sind erfindungsgemäß wenigstens die von Bruteiern umgebenden Bruteier von einer rohrförmig angeordneten Trennwand umgeben, so dass sich wenigstens diese Bruteier zwischen vier im Querschnitt einen viereckigen Raum begrenzenden Trennwänden befinden. Das bedeutet, dass die am Rand angeordneten Bruteier nach außen keine Trennwand aufweisen müssen.

Die elektromagnetischen Wellen besitzen nach der Weiterbildung des Patentanspruchs 2 Wellenlängen im Bereich jeweils einschließlich von 300 nm bis 1.000 nm.

Das Datenverarbeitungssystem ist nach der Weiterbildung des Patentanspruchs 3 ein die Eiabbilder nach dem mittleren Grauwert oder dem mittleren Farbwert klassifizierendes Datenverarbeitungssystem. Das kann entsprechend des RGB- oder des HSV-Farbraum erfolgen. Der RGB-Farbraum ist bekannterweise ein additiver Farbraum, der das additive Mischen dreier Grundfarben (Rot, Grün, Blau) nachbildet. Beim HSV-Farbraum ist bekannterweise der Farbort einer Farbe mit den Koordinaten Farbwert (hue), Farbsättigung (saturation) und Hellwert (value) definiert.

Das Datenverarbeitungssystem kann nach der Weiterbildung des Patentanspruchs 3 ferner ein die Eiabbilder nach dem mittleren Spektrum oder mehreren Einzelspektren klassifizierendes Datenverarbeitungssystem sein, wobei jedes Spektrum durch n Werte im Bereich jeweils einschließlich von 300 nm bis 1.000 nm gekennzeichnet ist.

In Transportrichtung nach der Einrichtung zur Untersuchung von Bruteiern ist nach der Weiterbildung des Patentanspruchs 4 wenigstens eine mindestens ein Brutei transportierende Vorrichtung angeordnet, welche derart ausgebildet ist, das jeweilige Brutei entsprechend des Ergebnisses der Untersuchung und damit der Eigenschaft einer der Eigenschaft zugeordneten Aufnahme zuzuführen. Weiterhin sind die Einrichtung zur Untersuchung und die Vorrichtung zum Transport des Bruteies mit dem Datenverarbeitungssystem verbunden, so dass die Einrichtung zur Untersuchung von Bruteiern, die das Brutei transportierende Vorrichtung und das Datenverarbeitungssystem eine Einrichtung zur Bewertung und Sortierung von Bruteiern nach den sich entwickelnden Embryos ist.

Der Träger ist erfindungsgemäß ein durchgängiges oder aus einzelnen Segmenten bestehendes Transportband mit den Trennwänden, welches weiterhin wenigstens im Bereich eines Bruteies eine Öffnung besitzt oder wenigstens im Bereich des Bruteies aus einem für die elektromagnetischen Wellen transparenten Material besteht.

Bruteier sind nach der Weiterbildung des Patentanspruchs 5 in Gruppen mit mehreren beabstandet zueinander angeordneten Reihen von Bruteiern auf dem Transportband angeordnet. In Transportrichtung nach der Einrichtung zur Untersuchung von Bruteiern befindet sich zur Identifizierung von unbefruchteten Bruteiern, zur Identifizierung von Bruteiern mit abgestorbenen Embryonen, zur Identifizierung von stark keimbelasteten Bruteiern und/oder zur Identifizierung des Geschlechts des sich entwickelnden Embryos der Gruppe von Bruteiern wenigstens eine die Bruteier entsprechend ihrer Identifikation transportierende Vorrichtung. Diese besitzt Halter entsprechend der Anzahl und der Position der Bruteier der Gruppe. Darüber hinaus sind diese Vorrichtung und die Halter mit dem Datenverarbeitungssystem verbunden, so dass die sortierten Bruteier einer Gruppe zu wenigstens einer Transporteinrichtung und/oder einem Behälter zugeführt werden.

Dem Transportband ist nach der Weiterbildung des Patentanspruchs 6 eine Bruteier zuführende Einrichtung vorgeordnet, die als Förderband in nacheinander angeordneten Reihen angeordnete Eihalter und/oder Bruthorden mit einer bestimmten Anzahl von Bruteiern (2) besitzt. Das Förderband und das als Förderband ausgebildete Transportband sind so zueinander positioniert, dass die Bruteier von der zuführenden Einrichtung mit einer Vorrichtung abgehoben und auf das als Förderband ausgebildete Transportband in die mittels Trennwänden ausgebildeten Kammern überführt werden.

Die Quelle für elektromagnetische Wellen befindet sich nach der Weiterbildung des Patentanspruchs 7 zwischen den Antriebstrommeln des als Förderband ausgebildeten Transportbandes.

Ein weiterer Aspekt der Erfindung ist die Verwendung der erfindungsgemäßen Einrichtung zur Untersuchung von Bruteiern ab dem fünften Bebrütungstag, ab dem zehnten Bebrütungstag und/oder ab dem sechzehnten Bebrütungstag.

Eine Ausführungsform einer Einrichtung kann eine Einrichtung zur Untersuchung von Bruteiern ab dem sechzehnten Bebrütungstag sein, wobei
- ein Träger mit beabstandet zueinander angeordneten Bruteiern zwischen einer Quelle für elektromagnetische Wellen und einer Hyperspektralkamera angeordnet ist,
- der Träger Ausschnitte zur Platzierung der Bruteier und lichtdichten Ankopplung der elektromagnetischen Wellen besitzt,
- sich zwischen den Bruteiern Trennwände befinden,
- die Quelle für elektromagnetische Wellen und die Hyperspektralkamera mit einem Datenverarbeitungssystem verbunden sind, welches aus dem Abbild der Hyperspektralkamera die Eiabbilder ermittelt und dem jeweiligen Eiabbild hyperspektrale Daten und die Position des jeweiligen Bruteies auf dem Träger zuordnet.

Ein Ausführungsbeispiel der Erfindung ist in den Zeichnungen jeweils prinzipiell dargestellt und wird im Folgenden näher beschrieben.

Es zeigen:
- Fig. 1: eine Einrichtung zur Untersuchung von Bruteiern,
- Fig. 2: ein Träger in einer Draufsicht,
- Fig. 3: eine Einrichtung zur Bewertung und Sortierung von Bruteiern und
- Fig. 4: eine Einrichtung zur Bewertung und Sortierung von Bruteiern nach dem Geschlecht des Embryos im Brutei.

Eine Einrichtung 1 zur Untersuchung von Bruteiern 2 besteht im Wesentlichen einem Träger 3 mit Bruteiern 2, einer Quelle 4 für elektromagnetische Wellen, einer Hyperspektralkamera 5 und einem Datenverarbeitungssystem 6.

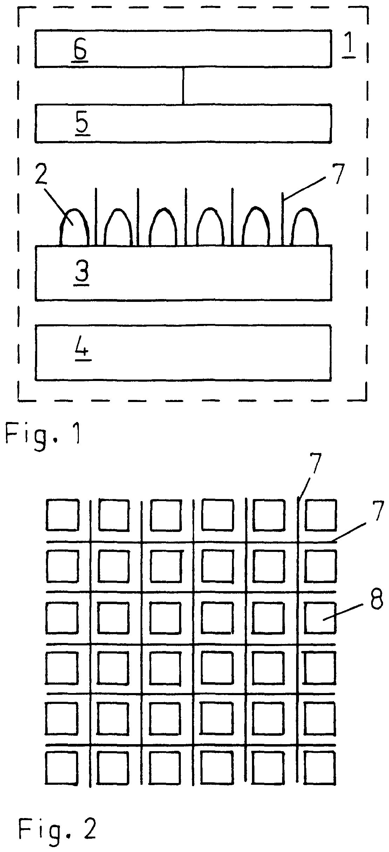

Die Fig. 1 zeigt eine Einrichtung 1 zur Untersuchung von Bruteiern 2 in einer prinzipiellen Darstellung.

Zur Untersuchung von Bruteiern 2 sind auf dem Träger 3 Bruteier 2 beabstandet zueinander angeordnet. Der Träger 3 besitzt dazu Ausschnitte zur Platzierung der Bruteier 2 und lichtdichten Ankopplung elektromagnetischer Wellen.

Die Fig. 2 zeigt einen Träger 3 in einer prinzipiellen Draufsicht.

Zwischen den Bruteiern 2 befinden sich Trennwände 7. Bis wenigstens auf die am Rand des Trägers 3 angeordneten Bruteier 2 sind Bruteier 2 von wenigstens einer rohrförmig angeordneten Trennwand 7 umgeben. Bruteier 2 am Rand sind wenigstens nur voneinander abgetrennt. Die Fig. 2 zeigt dazu vereinfacht nur Plätze 8 für jeweils ein Brutei 2.

Der Träger 3 ist zur Untersuchung zwischen der Quelle 4 für elektromagnetische Wellen mit Wellenlängen im Bereich jeweils einschließlich von 300 nm bis 1.000 nm und der Hyperspektralkamera 5 angeordnet. Diese sind weiterhin mit dem Datenverarbeitungssystem 6 verbunden, welches aus dem Abbild der Hyperspektralkamera 5 die Eiabbilder ermittelt und dem jeweiligen Eiabbild hyperspektrale Daten und die Position des jeweiligen Bruteies 2 auf dem Träger 3 zuordnet. Es erfolgt eine Klassifizierung, wobei die Eiabbilder
- nach dem mittleren Grauwert oder dem mittleren Farbwert und/oder
- nach dem mittleren Spektrum oder mehreren Einzelspektren, wobei jedes Spektrum durch n Werte im Bereich jeweils einschließlich von 300 nm bis 1.000 nm gekennzeichnet ist, klassifiziert werden.

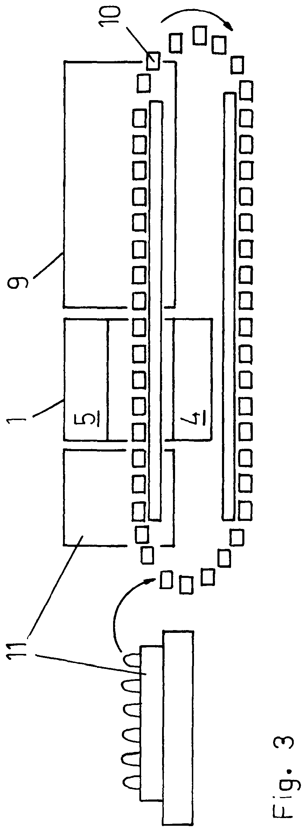

Die Fig. 3 zeigt eine Einrichtung zur Bewertung und Sortierung von Bruteiern 2 in einer prinzipiellen Darstellung.

In Transportrichtung nach der Einrichtung 1 zur Untersuchung von Bruteiern 2 ist in einer Ausführungsform zur Ausbildung einer Einrichtung zur Bewertung und Sortierung von Bruteiern 2 wenigstens eine mindestens ein Brutei 1 transportierende Vorrichtung 12 als ein Bestandteil einer Einrichtung 9 zur Sortierung angeordnet, die das jeweilige Brutei 2 entsprechend des Ergebnisses der Untersuchung und damit der Eigenschaft einer der Eigenschaft zugeordneten Aufnahme zuführt. Der Träger 3 ist dazu ein durchgängiges oder aus einzelnen Segmenten bestehendes Transportband 10 mit den Trennwänden 7. Das Transportband 10 besitzt wenigstens im Bereich eines Bruteies 2 eine Öffnung oder besteht wenigstens im Bereich des Bruteies 2 aus einem für die elektromagnetischen Wellen transparenten Material. Dem Transportband 10 ist eine Bruteier 2 zuführende Einrichtung 11 vorgeordnet, die als Förderband in nacheinander angeordneten Reihen angeordnete Eihalter und/oder Bruthorden mit einer bestimmten Anzahl von Bruteiern 2 besitzen kann. Das Förderband und das als Förderband ausgebildete Transportband 10 sind so zueinander positioniert, dass die Bruteier 2 von der zuführenden Einrichtung 11 mit einer Vorrichtung abgehoben und auf das als Förderband ausgebildete Transportband 11 in die mittels Trennwänden 7 ausgebildeten Kammern überführt werden. Die Antriebe der Förderbänder, die das Brutei 2 transportierende Vorrichtung 12 sind mit dem Datenverarbeitungssystem 6 verbunden.

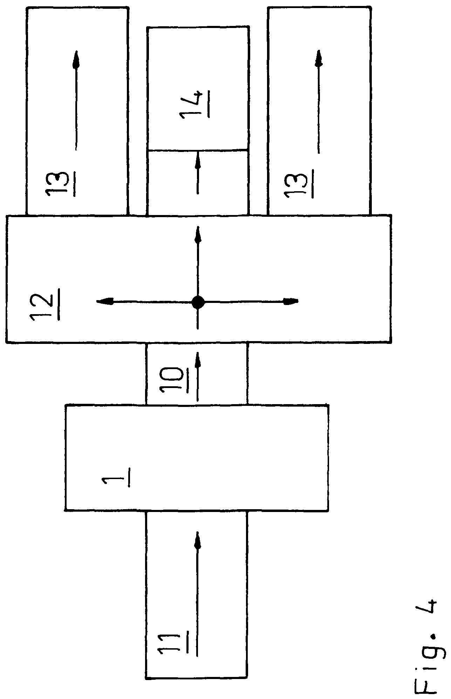

Die Fig. 4 zeigt eine Einrichtung zur Bewertung und Sortierung von Bruteiern 2 nach dem Geschlecht des Embryos im Brutei 2.

In dieser Ausführungsform einer Einrichtung zur Bewertung und Sortierung mit der Einrichtung 1 zur Untersuchung können die Bruteier 2 entsprechend des Geschlechts des Embryos im Brutei 2 sortiert werden. Der Einrichtung 1 zur Untersuchung ist die Bruteier 2 zuführende Einrichtung 11 vorgeordnet, die als Förderband die in nacheinander angeordneten Reihen angeordnete Eihalter besitzt. Diesem ist das Transportband 10 nachgeordnet, welches wenigstens im Bereich eines Bruteies 2 eine Öffnung besitzt oder wenigstens im Bereich des Bruteies 2 aus einem für die elektromagnetischen Wellen transparenten Material besteht. Das Förderband und das als Förderband ausgebildete Transportband 10 sind so zueinander positioniert, dass die Bruteier 2 von der zuführenden Einrichtung 11 mit einer Vorrichtung abgehoben und auf das als Förderband ausgebildete Transportband 11 in die mittels Trennwänden 7 ausgebildeten Kammern überführt werden. Die Bruteier 2 sind dazu in Gruppen mit mehreren beabstandet zueinander angeordneten Reihen von Bruteiern 2 auf dem Transportband angeordnet. Mittels der Einrichtung 1 zur Untersuchung werden die Bruteier 2 einer Gruppe mit elektromagnetischen Wellen der Quelle 4 für elektromagnetische Wellen beaufschlagt und durch die Hyperspektralkamera 5 die Bruteier 2 der Gruppe abgebildet. In Transportrichtung nach der Einrichtung 1 zur Untersuchung von Bruteiern 2 einer Gruppe zur Identifizierung von unbefruchteten Bruteiern, zur Identifizierung von abgestorbenen Embryonen, zur Identifizierung von stark keimbelasteten Bruteiern und/oder zur Identifizierung des Geschlechts des sich entwickelnden Embryos der Gruppe von Bruteiern 2 ist wenigstens die die Bruteier 2 entsprechend ihrer Identifikation transportierende Vorrichtung 12 angeordnet. Diese kann Halter entsprechend der Anzahl und der Position der Bruteier 2 der Gruppe besitzen, wobei die Vorrichtung 12 und die Halter mit dem Datenverarbeitungssystem 6 verbunden. Damit können die sortierten Bruteier 2 einer Gruppe zu Transporteinrichtungen 13 und wenigstens einem Behälter 14 zugeführt werden.

Dazu ist eine Transporteinrichtung 13 für weibliche sich entwickelnde Embryonen in Bruteiern 2 und eine andere Transporteinrichtung 13 für männliche sich entwickelnde Embryonen in Bruteiern vorhanden. Der Behälter 14 nimmt insbesondere unbefruchtete Bruteier 2, Bruteier 2 mit abgestorbenen Embryonen und stark keimbelastete Bruteier 2 auf. Die Antriebe der Transporteinrichtungen 13 sind mit dem Datenverarbeitungssystem 6 verbunden.

## Patentansprüche

1. Einrichtung (1) zur Untersuchung von Bruteiern (2), mit einem Träger (3) auf dem sich, wenn in Benutzung, beabstandet zueinander angeordnete Bruteier (2) befinden und welcher zwischen einer Quelle (4) für elektromagnetische Wellen und einer Hyperspektralkamera (5) angeordnet ist, wobei der Träger (3) Ausschnitte zur Platzierung der Bruteier (2) und lichtdichten Ankopplung der elektromagnetischen Wellen besitzt, und wobei sich zwischen den Bruteiern (2) Trennwände (7) befinden,
**dadurch gekennzeichnet dass** auf dem Träger (3) wenigstens die von Bruteiern (2) umgebenden Bruteier (2) von einer rohrförmig angeordneten Trennwand (7) umgeben sind, so dass sich wenigstens diese Bruteier (2) zwischen vier im Querschnitt einen viereckigen Raum begrenzenden Trennwänden (7) befinden und dass die Quelle (4) für elektromagnetische Wellen und die Hyperspektralkamera (5) mit einem Datenverarbeitungssystem (6) verbunden sind, welches derart ausgebildet ist, aus dem Abbild der Hyperspektralkamera (5) die Eiabbilder zu ermitteln und dem jeweiligen Eiabbild hyperspektrale Daten und die Position des jeweiligen Bruteies (2) auf dem Träger (3) zuzuordnen, wobei der Träger (3) ein durchgängiges oder aus einzelnen Segmenten bestehendes Transportband (10) mit den Trennwänden (7) ist, und dass das Transportband (10) wenigstens im Bereich eines Bruteies (2) eine Öffnung besitzt oder wenigstens im Bereich des Bruteies (2) aus einem für die elektromagnetischen Wellen transparenten Material besteht.

2. Einrichtung nach Patentanspruch 1, **dadurch gekennzeichnet, dass** die elektromagnetischen Wellen Wellenlängen im Bereich jeweils einschließlich von 300 nm bis 1.000 nm besitzen.

3. Einrichtung nach Patentanspruch 1, **dadurch gekennzeichnet, dass** das Datenverarbeitungssystem (6)
- ein die Eiabbilder nach dem mittleren Grauwert klassifizierendes Datenverarbeitungssystem oder
- ein die Eiabbilder nach dem mittleren Farbwert klassifizierendes Datenverarbeitungssystem (6) oder
- ein die Eiabbilder nach dem mittleren Spektrum klassifizierendes Datenverarbeitungssystem (6) ist, wobei jedes Spektrum durch n Werte im Bereich jeweils einschließlich von 300 nm bis 1000 nm gekennzeichnet ist, oder
- ein die Eiabbilder nach mehreren Einzelspektren klassifizierendes Datenverarbeitungssystem (6) ist, wobei jedes Spektrum durch n Werte im Bereich jeweils einschließlich von 300 nm bis 1000 nm gekennzeichnet ist.

4. Einrichtung nach Patentanspruch 1, **dadurch gekennzeichnet, dass** in Transportrichtung nach der Einrichtung (1) zur Untersuchung von Bruteiern (2) wenigstens eine mindestens ein Brutei (2) transportierende Vorrichtung (12) angeordnet ist, welche derart ausgebildet ist, das jeweilige Brutei (2) entsprechend des Ergebnisses der Untersuchung und damit der Eigenschaft einer der Eigenschaft zugeordneten Aufnahme zuzuführen, und dass die Einrichtung (1) zur Untersuchung und die Vorrichtung zum Transport des Bruteies (2) mit dem Datenverarbeitungssystem (6) verbunden sind, so dass die Einrichtung (1) zur Untersuchung von Bruteiern (2), die das Brutei (2) transportierende Vorrichtung (12) und das Datenverarbeitungssystem (1) eine Einrichtung (9) zur Bewertung und Sortierung von Bruteiern (2) nach den sich entwickelnden Embryos ist.

5. Einrichtung nach Patentanspruch 1, **dadurch gekennzeichnet, dass** Bruteier (2) in Gruppen mit mehreren beabstandet zueinander angeordneten Reihen von Bruteiern (2) auf dem Transportband (10) anordenbar sind, dass sich in Transportrichtung nach der Einrichtung (1) zur Untersuchung von Bruteiern zur Identifizierung von unbefruchteten Bruteiern (2), zur Identifizierung von Bruteiern (2) mit abgestorbenen Embryonen, zur Identifizierung von stark keimbelasteten Bruteiern (2) und/oder zur Identifizierung des Geschlechts des sich entwickelnden Embryos der Gruppe von Bruteiern (2) wenigstens eine die Bruteier (2) entsprechend ihrer Identifikation transportierende Vorrichtung (12) angeordnet ist, dass die Vorrichtung (12) Halter entsprechend der Anzahl und der Position der Bruteier (2) der Gruppe besitzt, dass die Vorrichtung (12) und die Halter mit dem Datenverarbeitungssystem (6) verbunden sind, so dass die sortierten Bruteier (2) einer Gruppe zu wenigstens einer Transporteinrichtung (13) und/oder einem Behälter (14) zugeführt werden.

6. Einrichtung nach den Patentansprüchen 1 und 5, **dadurch gekennzeichnet, dass** dem Transportband (10) eine Bruteier (2) zuführende Einrichtung (11) vorgeordnet ist, dass die Bruteier (2) zuführende Einrichtung (11) als
Förder-band in nacheinander angeordneten Reihen angeordnete Eihalter und/oder Bruthorden mit einer bestimmten Anzahl von Bruteiern (2) besitzt, dass das Förderband und das als Förderband ausgebildete Transportband so zueinander positioniert sind, dass die Bruteier (2) von der zuführenden Einrichtung mit einer Vorrichtung abgehoben und auf das als Förderband ausgebildete Transport-band in die mittels Trennwänden (7) ausgebildeten Kammern überführt werden.

7. Einrichtung nach Patentanspruch 1, **dadurch gekennzeichnet, dass** sich die Quelle (4) für elektromagnetische Wellen zwischen den Antriebstrommeln des als Förderband ausgebildeten Transportbandes (10) befindet.

8. Verwendung einer Einrichtung (1) zur Untersuchung von Bruteiern (2) nach Patentanspruch 1, **dadurch gekennzeichnet, dass** die Einrichtung (1) eine Einrichtung (1) zur Untersuchung von Bruteiern (2) ab dem fünften Bebrütungstag, ab dem zehnten Bebrütungstag und/oder ab dem sechzehnten Bebrütungstag ist.

## Claims

1. Device (1) for examining hatching eggs (2), comprising a carrier (3) on which, when in use, hatching eggs (2) arranged at a distance from one another are located, and which
is arranged between a source (4) for electromagnetic waves and a hyperspectral camera (5), the carrier (3) having cutouts for the placement of the hatching eggs (2) and for the light-proof coupling of the electromagnetic waves, and
partition walls (7) being located between the hatching eggs (2),
**characterised in that,** on the carrier (3), at least the hatching eggs (2) surrounded by hatching eggs (2) are surrounded by a partition wall (7) arranged in a tubular manner, such that at least these hatching eggs (2) are located between four partition walls (7) which delimit a quadrilateral space in cross section, **and in that** the source (4) for electromagnetic waves and the hyperspectral camera (5) are connected to a data processing system (6), which is designed to determine the egg images from the image from the hyperspectral camera (5) and to assign hyperspectral data and the position of the particular hatching egg (2) on the carrier (3) to each particular egg image, the carrier (3) being a transport belt (10) which is continuous or consists of individual segments and which comprises the partition walls (7), **and in that** the transport belt (10) has an opening at least in the region of a hatching egg (2) or consists of a material transparent to the electromagnetic waves at least in the region of the hatching egg (2).

2. Device according to claim 1, **characterised in that** the electromagnetic waves have wavelengths in the range respectively including from 300 nm to 1000 nm.

3. Device according to claim 1, **characterised in that** the data processing system (6) is
- a data processing system that classifies the egg images according to the average grey scale value, or
- a data processing system (6) that classifies the egg images according to the average colour value, or
- a data processing system (6) that classifies the egg images according to the average spectrum, each spectrum being **characterised by** n values in the range respectively including from 300 nm to 1000 nm, or
- a data processing system (6) that classifies the egg images according to a plurality of individual spectra, each spectrum being **characterised by** n values in the range respectively including from 300 nm to 1000 nm.

4. Device according to claim 1, **characterised in that** at least one apparatus (12) which transports at least one hatching egg (2) is arranged, in the transport direction, downstream of the device (1) for examining hatching eggs (2), which apparatus is designed to supply the particular hatching egg (2) according to the result of the examination and thus according to the property to a receptacle assigned to the property, **and in that** the device (1) for examining and the apparatus for transporting the hatching egg (2) are connected to the data processing system (6) such that the device (1) for examining hatching eggs (2), the apparatus (12) which transports the hatching egg (2) and the data processing system (1) constitute a device (9) for assessing and sorting hatching eggs (2) according to the developing embryo.

5. Device according to claim 1, **characterised in that** hatching eggs (2) can be arranged on the transport belt (10) in groups having a plurality of rows of hatching eggs (2) arranged at a distance from one another, **in that** at least one apparatus (12) which transports the hatching eggs (2) according to their identification is arranged, in the transport direction, downstream of the device (1) for examining hatching eggs so as to identify unfertilised hatching eggs (2), so as to identify hatching eggs (2) containing dead embryos, so as to identify highly germ-contaminated hatching eggs (2) and/or so as to identify the sex of the developing embryo of the group of hatching eggs (2), **in that** the apparatus (12) has holders corresponding to the number and position of the hatching eggs (2) of the group, **and in that** the apparatus (12) and the holders are connected to the data processing system (6) such that the sorted hatching eggs (2) of a group are fed to at least one transport device (13) and/or container (14).

6. Device according to claims 1 and 5, **characterised in that** a device (11) which supplies hatching eggs (2) is arranged upstream of the transport belt (10), **in that** the device (11) which supplies hatching eggs (2) has, as a conveyor belt, egg holders and/or incubators which contain a certain number of hatching eggs (2) and are arranged in rows arranged one behind another, **in that** the conveyor belt and the transport belt designed as a conveyor belt are positioned relative to one another such that the hatching eggs (2) are lifted off the supply device by means of an apparatus and transferred, on the transport belt designed as a conveyor belt, into the chambers formed by means of partition walls (7).

7. Device according to claim 1, **characterised in that** the source (4) for electromagnetic waves is located between the drive pulleys of the transport belt (10) designed as a conveyor belt.

8. Use of a device (1) for examining hatching eggs (2) according to claim 1, **characterised in that** the device (1) is a device (1) for examining hatching eggs (2) from the fifth incubation day, from the tenth incubation day and/or from the sixteenth incubation day.

## Revendications

1. Appareil (1) permettant d'examiner des oeufs à couver (2), comportant un support (3) sur lequel se trouvent des oeufs à couver (2) espacés les uns des autres lors de l'utilisation et lequel
est disposé entre une source (4) d'ondes électromagnétiques et une caméra hyperspectrale (5), le support (3) présentant des découpes pour le placement des oeufs à couver (2) et le couplage des ondes électromagnétiques étanche à la lumière, et
des cloisons (7) se trouvant entre les oeufs à couver (2),
**caractérisé en ce que**, sur le support (3), au moins les oeufs à couver (2) entourés par des oeufs à couver (2) sont entourés par une cloison (7) tubulaire, de sorte qu'au moins lesdits oeufs à couver (2) se trouvent entre quatre cloisons (7) délimitant un espace quadrangulaire en section transversale, **et en ce que** la source (4) d'ondes électromagnétiques et la caméra hyperspectrale (5) sont connectées à un système de traitement de données (6) configuré pour déterminer les images d'oeuf à partir de l'image de la caméra hyperspectrale (5) et pour associer des données hyperspectrales et la position de l'oeuf à couver (2) respectif sur le support (3) à l'image d'oeuf respective, le support (3) étant une bande transporteuse (10) continue ou constituée de segments individuels et comportant les cloisons (7), **et en ce que** la bande transporteuse (10) présente une ouverture au moins dans la zone d'un oeuf à couver (2) ou est constituée d'un matériau transparent aux ondes électromagnétiques au moins dans la zone de l'œuf à couver (2).

2. Appareil selon la revendication 1, **caractérisé en ce que** les ondes électromagnétiques présentent des longueurs d'onde dans la plage entre 300 nm et 1 000 nm respectivement y compris.

3. Appareil selon la revendication 1, **caractérisé en ce que** le système de traitement de données (6) est
- un système de traitement de données classifiant les images d'oeuf selon la valeur de gris moyenne ou
- un système de traitement de données (6) classifiant les images d'oeuf selon la valeur de couleur moyenne ou
- un système de traitement de données (6) classifiant les images d'oeuf selon le spectre moyen, chaque spectre étant **caractérisé par** n valeurs dans la plage entre 300 nm et 1 000 nm respectivement y compris, ou
- un système de traitement de données (6) classifiant les images d'oeuf selon plusieurs spectres individuels, chaque spectre étant **caractérisé par** n valeurs dans la plage entre 300 nm et 1 000 nm respectivement y compris.

4. Appareil selon la revendication 1, **caractérisé en ce qu'**au moins un dispositif (12) transportant au moins un oeuf à couver (2) est disposé dans la direction de transport après l'appareil (1) permettant d'examiner des oeufs à couver (2), lequel dispositif est conçu pour acheminer l'œuf à couver (2) respectif en fonction du résultat de l'examen et donc de la propriété à une prise associée à la propriété, **et en ce que** l'appareil (1) permettant d'examiner et le dispositif permettant de transporter l'œuf à couver (2) sont connectés au système de traitement de données (6), de sorte que l'appareil (1) permettant d'examiner des oeufs à couver (2), le dispositif (12) transportant l'œuf à couver (2) et le système de traitement de données (1) sont un appareil (9) permettant d'évaluer et de trier des oeufs à couver (2) en fonction des embryons en développement.

5. Appareil selon la revendication 1, **caractérisé en ce que** des oeufs à couver (2) peuvent être disposés sur la bande transporteuse (10) en groupes comportant plusieurs rangées d'oeufs à couver (2) espacées les unes des autres, **en ce qu'**au moins un dispositif (12) transportant les oeufs à couver (2) conformément à leur identification est disposé dans la direction de transport après l'appareil (1) permettant d'examiner des oeufs à couver pour l'identification d'oeufs à couver (2) non fécondés, pour l'identification d'oeufs à couver (2) comportant des embryons morts, pour l'identification d'oeufs à couver (2) fortement contaminés par des germes et/ou pour l'identification du sexe de l'embryon en développement du groupe d'oeufs à couver (2), **en ce que** le dispositif (12) présente des soutiens correspondant au nombre et à la position des oeufs à couver (2) du groupe, **en ce que** le dispositif (12) et les soutiens sont connectés au système de traitement de données (6), de sorte que les oeufs à couver (2) triés d'un groupe sont acheminés vers au moins un appareil de transport (13) et/ou un récipient (14).

6. Appareil selon les revendications 1 et 5, **caractérisé en ce qu'**un appareil (11) acheminant des oeufs à couver (2) est disposé en amont de la bande transporteuse (10), **en ce que** l'appareil (11) acheminant des oeufs à couver (2) présente des soutiens d'oeuf et/ou des plateaux d'incubation comportant un nombre déterminé d'oeufs à couver (2) et disposés en rangées successives en tant que convoyeur à bande, **en ce que** le convoyeur à bande et la bande transporteuse réalisée en tant que convoyeur à bande sont positionnés l'un par rapport à l'autre de telle sorte que les oeufs à couver (2) sont soulevés de l'appareil d'acheminement au moyen d'un dispositif et sont transférés sur la bande transporteuse réalisée en tant que convoyeur à bande dans les chambres réalisées au moyen de cloisons (7).

7. Appareil selon la revendication 1, **caractérisé en ce que** la source (4) d'ondes électromagnétiques se trouve entre les tambours d'entraînement de la bande transporteuse (10) réalisée en tant que convoyeur à bande.

8. Utilisation d'un appareil (1) permettant d'examiner des oeufs à couver (2) selon la revendication 1, **caractérisée en ce que** l'appareil (1) est un appareil (1) permettant d'examiner des oeufs à couver (2) à partir du cinquième jour d'incubation, à partir du dixième jour d'incubation et/ou à partir du seizième jour d'incubation.
